# EUROPÄISCHE PATENTSCHRIFT

(11) **EP 2 651 479 B1**
(45) Veröffentlichungstag und Bekanntmachung des Hinweises auf die Patenterteilung: **30.05.2018**
(21) Anmeldenummer: 11793655.9
(22) Anmeldetag: 21.11.2011
(51) Int. Cl.: A61M 11/02, A61M 16/06, A61M 15/08

(54) **THERAPIEGERÄT**
THERAPY APPLIANCE
APPAREIL DE THÉRAPIE

(30) Priorität: 13.12.2010 DE 102010054361
(43) Veröffentlichungstag der Anmeldung: 23.10.2013
(73) Patentinhaber: Nli GmbH, 35043 Marburg (DE)
(72) Erfinder: KEOHLER, Ulrich, 35043 Marburg (DE); GROSS, Volker, 35453 Wettenberg (DE); SOHRABI, Keywan, Ali, 35392 Glessen (DE)
(74) Vertreter: Walkenhorst, Andreas
(86) Internationale Anmeldenummer: PCT/EP2011/005849
(87) Internationale Veröffentlichungsnummer: WO 2012/079684

(56) Entgegenhaltungen:
- WO-A2-2006/102345
- US-A1- 2004 210 153
- US-A1- 2005 229 927
- US-A1- 2005 268 916
- US-A1- 2010 074 881
- David E. Geller ET AL: "The I-neb Adaptive Aerosol Delivery System Enhances Delivery of [alpha]1-Antitrypsin with Controlled Inhalation", Journal of aerosol medicine and pulmonary drug delivery, vol. 23, no. s1, 1 April 2010 (2010-04-01) , pages S-55, XP055349827, US ISSN: 1941-2711, DOI: 10.1089/jamp.2009.0793

## Beschreibung

Die Erfindung betrifft ein Therapiegerät, insbesondere für eine individualisierte nasale Langzeit-Inhalationstherapie, mit einem Beatmungsschlauch, an den eingangsseitig ein mit einem Medikamentenbehälter verbundener Aerosolerzeuger angeschlossen ist, der mit einer Steuerungseinheit verbunden ist.

Chronische Lungenerkrankungen wie beispielsweise Asthma bronchiale oder chronisch Obstruktive Lungenerkrankung (COPD) werden bei der erwachsenen Bevölkerung in Deutschland auf 10 bis 15 % geschätzt. Weltweit stellen diese Erkrankungen gegenwärtig die vierthäufigste Todesursache dar. Für die nächsten Jahrzehnte ist ein weiterer Anstieg dieser chronischen Lungenerkrankungen zu erwarten. Laut WHO wird die COPD im Jahre 2020 unter den häufigsten Todesursachen auf den 3. Platz vorrücken.

Die Inhalationstherapie spielt eine zentrale Rolle bei vielen Patienten mit Mukoviszidose, Asthma bronchiale und COPD. Durch die inhalative Gabe von Steroiden, Anticholinergika und ß-Sympathomimetika können, im Vergleich zur oralen Gabe, die Nebenwirkungen reduziert und bessere Therapieerfolge erzielt werden.

Viele Patienten führen täglich eine oder mehrere Inhalationen durch, die allerdings aufgrund von falscher Anwendung zu keiner, oder zu einer verminderten Wirkung führen. Gleichzeitig werden mitunter mehrere Dosen inhaliert, was wiederum oft eine Überdosierung nach sich zieht und besonders bei Patienten mit Herz-Kreis-Iauf-Vorschädigungen zu einer höheren Sterblichkeit führen kann.

Die DE 10 2008 050 218 beschreibt ein System und ein Verfahren zur Applikation inhalierbarer Stoffe in eine Lunge, insbesondere in eine mit einem Beatmungsgerät verbundene Tierlunge. Während der Entwicklungsphase eines neuen Wirkstoffes ist dieser in der Regel nur in geringen Mengen vorhanden, so dass der effizienten Applikation während der Versuchsphase besondere Bedeutung zukommt. So kommt dieses System als Prüfsystem und Prüfverfahren während der Entwicklungsphase neuer Wirkstoffe zum Einsatz.

Eine Apparatur und ein Verfahren zur Optimierung einer Dosisabscheidung bei einer inhalatorischen Medikamentenapplikation wird in der EP 2 085 105 beschrieben. Die Apparatur ermittelt zunächst das individuelle Atemzugvolumen eines Patienten um die vorgesehene Medikamentendosisabscheidung in einen vorbestimmten Lungenbereich zu optimieren. Hierzu wird die Medikamenten-Partikel-Konzentration in einer Medikamenten-Aerosol-Menge an das ermittelte Atemzugvolumen angepasst und über ein Mundstück inhaliert. Eine Erfassungseinrichtung signalisiert das Erreichen des zu inhalierenden Atemzugvolumens der Medikamenten-Aerosol-Menge, das für die vorgesehene Medikamentendosisabscheidung in dem vorbestimmten Lungenbereich mit einem Atemzug notwendig ist.

Aus der US 2004/210153 A1 ist ein Therapiergerät zur Verabreichung von Medikamenten durch nasale Inhalation bekannt, bei dem ein eingansgseitig an eine Anzahl von Medikamentenbehältern verbundener Beatmungsschlauch endseitig an einen nasalen Applikator angeschlossen ist.

Bei der COPD kommt es pathophysiologisch durch die entzündlichen Veränderungen der kleinen Atemwege zu einer Obstruktion derselben. Damit ist der gewünschte therapeutische Effekt mit konventionellen medikamentösen Inhalationsverfahren nur bedingt möglich. Problematisch bei der bisherigen Therapie der Erkrankung ist, dass gängige Medikamente (Betamimetika, Steroide) aufgrund der Obstruktion oft nicht die peripheren Atemwege erreichen. Des Weiteren treten die Beschwerden gehäuft nachts auf (Hypoventilation, chronobiologische Rhythmik).

Die Therapietreue und Akzeptanz der Aerosoltherapie durch die Patienten könnte erhöht werden, wenn es gelingt die Zeit der Therapie von einem aktiven Vorgang am Tage in einen passiven Vorgang in der Nacht zu verschieben.

Der Erfindung liegt nunmehr die Aufgabe zugrunde, ein Therapiegerät der oben genannten Art bereitzustellen, das eine besonders gezielte und kontrollierte Applizierung oder Verabreichung von Medikamenten in die Atemwege, insbesondere in die Lunge, eines Patienten ermöglicht.

Diese Aufgabe wird erfindungsgemäß gelöst durch die Merkmale des Anspruchs 1.

Vorteilhafte Ausgestaltungen der Erfindung sind Gegenstand der Unteransprüche.

Die Erfindung geht von der Überlegung aus, dass eine besonders gezielte und kontrollierte Applizierung oder Verabreichung von Medikamenten in die Atemwege eines Patienten insbesondere durch eine konsequente Ausrichtung der Medikation auf eine nasale Inhalation unter Rückgriff auf die damit erreichbaren vergleichsweise langsamen Luft- oder Gasaustauschvorgänge erreichbar ist. Demgegenüber ist die Inhalationstherapie mit konventionellen Methoden bei Patienten mit schlechten Lungenfunktionswerten (z. B. bei schwer einstellbarem Asthma bronchiale) oft nicht mehr erfolgreich einsetzbar. Durch eine kontrollierte und druckunterstützte, nächtliche Applikation eines Aerosolwirkstoffs über die Nase kann die Therapie jedoch optimiert werden.

Mit der neuen Vorrichtung können auch neue Therapieansätze verfolgt werden. Dabei verwendet man eine individualisierte Langzeit-Inhalationstherapie mit individueller Dosis bei individuellem Zeitprofil. Dabei ermöglicht es diese Vorrichtung verschiedene Medikamente in definierten Konzentrationen zu definierten Zeitpunkten zu verabreichen. Dieses Konzept eignet sich z. B. für die kombinierte Behandlung mit Steroiden und Betamimetika bei Patienten mit COPD. Das Verfahren führt zu einer besseren Ausnutzung von Wirkstoffen bei geringeren Nebeneffekten. Die langsame Abgabe von Wirkstoffen über einen längeren Zeitraum (Low Dose - Long-Term) hat sich als sehr vorteilhaft erwiesen, da bei vielen Wirkstoffen ein gleichbleibender Wirkstoffspiegel im Blutplasma erwünscht ist. Mit der neuen Vorrichtung und dem neuen Verfahren kann eine solche "Low Dose - Long Time"- Inhalationstherapie durchgeführt werden.

Die Wirkung der nasalen Inhalation wird zusätzlich durch eine kontrollierte Beatmung verbessert.

Durch eine spezifische Bolusabgabe im Atemzyklus kann zusätzlich nach den Wirkorten unterschieden werden (peripher/zentral), je nachdem ob ein Aerosolbolus am Anfang oder am Ende der Inspiration verabreicht wird.

Die Inspirationszeit während einer Inhalation kann durch die kontrollierte Beatmung verlängert werden. Durch die langen Aufenthaltszeiten der Partikel in der Lunge können diese Partikel aufgrund von Sedimentations- und Diffusionsvorgängen besser deponiert und damit den Wirkstoffübergang in die Lunge erhöhen werden.

Vorzugsweise wird die künstliche Verlängerung der Inspirationszeit während der Inhalation nur im Schlaf durchgeführt, um vom Patienten nicht als unangenehm empfunden zu werden. Die Detektion der Schlafphasen kann beispielsweise durch Aktigraphie (Lagesensor) oder durch eine Auswertung der Herzfrequenzvariabilität (Pulsoximeter) erfolgen.

In vorteilhafter Ausführung ist der Aerosolerzeuger als Aerosolvernebler ausgeführt, der insbesondere durch Zumischung einer zuvor zerstäubten Medikamentendosis in einen Gas- oder Luftstrom das Aerosol erzeugt. Besonders vorteilhaft ist dabei ein regelbarer Düsen-Aerosol-Vernebler vorgesehen. Der Aerosolvernebler ist vorzugsweise derart ausgebildet, dass er durch die Wahl des Kompressordruckes und/oder die Verwendung bestimmter Filter, Aerosolpartikel mit einer Größe zwischen 0,1 und 10 µm erzeugen kann.

Um ein Medikament in Form eines Aerosols vernebelt über die Nase in die Atemwege und die Lunge zu applizieren, müssen die Aerosolpartikel zunächst den Nasentrakt passieren. Die Nase ist jedoch ein sehr effizienter Partikelfilter, der nur sehr kleine Partikel in die Lunge durchlässt. Durch die geeignete Wahl der Parti-kelgröße, die möglichst im Bereich zwischen 0,1 und 3 µm liegt (idealerweise bei 1,3 µm), wird sichergestellt, dass möglichst viele inhalierte Aerosolpartikel nicht durch das sehr effiziente Filtersystem der Nase herausgefiltert werden und somit in die Lunge inhaliert werden. Sollte es jedoch gewünscht sein, eine Deposition im Nasenrachenraum zu erreichen, so kann dies auf einfache Weise durch eine entsprechende Variation der Partikelgröße realisiert werden, vorzugsweise liegen hier die Partikelgrößen zwischen 3 µm und 10 µm.

Der Aerosolerzeuger ist erfindungsgemäß mit der Steuerungseinheit verbunden und wird durch diese nach einem vorgegebenen Aktivierungsmuster aktiviert. Zusätzlich begrenzt die Steuerungseinheit erfindungsgemäß den Inhalationsfluss während der Aerosolpartikelzufuhr, erfindungsgemäß auf 500 ml/s. In zusätzlicher erfindungsgemäßer Ausgestaltung regelt die Steuerungseinheit zudem das Inhalationsvolumen auf mehr als 0,3 l. Die solchermaßen bevorzugt vergleichsweise niedrig gehaltenen Inspirationflüsse wie oben angegeben tragen dazu bei, eine hohe Nasendeposition zu vermeiden. Das Inhalationsvolumen hingegen bewirkt eine hohe Effizienz der Aerosolapplikation in der Lunge.
Die Inhalationsgeschwindigkeit ist durch die nächtliche Spontanatmung eher gering, was der Deposition in der Lungenperipherie zugutekommt. Zusätzlich wird besonders vorteilhaft bei beatmeten Patienten der Inhalationsfluss während der Aerosol-Applikationsphasen entsprechend niedrig eingestellt oder/und durch eine geeignete Wahl von mechanischen Flussbegrenzern sichergestellt.
Die Steuerungseinrichtung aktiviert den Aerosolerzeuger, in vorteilhafter Weise abgestimmt auf das Atemmuster des Patienten, gemäß einem Aktivierungsmuster regelmäßiger, ganzzahliger Vielfacher des Atemmustertaktes des Patienten. Vorzugsweise erzeugt die Steuerungseinrichtung einen Aerosolbolus jeden dritten, fünften, siebten, oder achten Atemzug.
Gemäß einer bevorzugten Ausführungsform aktiviert die Steuerungseinrichtung den Aerosolerzeuger während der Expiration oder Ausatmung des Patienten zur Erzeugung eines Aerosolbolus in den Beatmungsschlauch. Dies bedeutet, dass der Beatmungsschlauch als Aerosolreservoir dient und während der Expiration des Patienten (welche vorteilhafterweise in der Art eines mehrstrangigen Systems in einem separaten Schlauch und am Beatmungsschlauch vorbei erfolgt) erneut gefüllt wird, um dann während der darauffolgenden Einatmung leer geatmet zu werden.

Der Beatmungsschlauch erfüllt damit die Funktion einer Applikationshilfe, eines so genannten Spacers. Die Aerosolpartikel werden im Spacer homogen vermischt und in der Schwebe gehalten. Zusätzlich erleichtert der Spacer das langsame Einatmen der Aerosolpartikel.

Durch die gezielte Kombination aus Partikelgröße, Inhalationsfluss und Inhalationsvolumen stellt das Therapiegerät nun einen Weg bereit, den nachteiligen Einfluss der Partikelfilterfunktion der Nase deutlich zu reduzieren und zugleich die Effizienz der Partikelabscheidung in der Lunge deutlich zu erhöhen.

In vorteilhafter Ausführung ist eine Beatmungsvorrichtung vorgesehen, die gemäß einem vorgegebenen Atemmuster dem Patienten über den Beatmungsschlauch während der Einatmung kontinuierlich Atemluft und/oder Sauerstoff zuführt. Das der Beatmungsvorrichtung vorgegebene Atemmuster ist dabei vorzugsweise abgestimmt auf den Sauerstoffbedarf des Patienten, welcher Vorzugsweise aus der Pulsoximetrie ermittelt wird.

In vorteilhafter Weise kommt im Rahmen dieser Beatmungsvorrichtung ein druckkontrolliertes Beatmungsgerät zum Einsatz. Das druckkontrollierte Beatmungsgerät ist vorteilhafterweise als CPAP (Continuous Positive Airway Pressure)-Gerät und/oder als BIPAP (Biphasic Positive Airway Pressure)-Gerät ausgestaltet. Durch die nicht-invasive Überdruck-Beatmung wird eine Verbesserung der Deposition des Aerosols in die Lungenperipherie erzielt. Der applizierte Überdruck erzeugt eine pneumatische Schienung der kleinen Atemwege und hält diese so offen, um so vor allem einen Zugang in obstruktive Bereiche der peripheren Lunge zu ermöglichen, wie sie beispielsweise bei COPD-Patienten zu finden sind.

Vorzugsweise ist das System mit mehreren Medikamentenbehältern ausgestattet und ermöglicht es eine auf den jeweiligen Wirkstoff abgestimmte Aerosolabgabemenge und ein abgestimmtes Zeitprofil zu erzeugen, um beispielsweise Bronchodilatatoren und anti-inflammatorische Wirkstoffe zu unterschiedlichen Zeiten und in unterschiedlichen Mengen zu applizieren.

Das Therapiegerät weist ferner in vorteilhafter Ausführung einen Sensor zur Lagebestimmung des Patienten auf. Damit kann z. B. bei der Langzeitinhalation in der Nacht festgestellt werden, ob der Patient schläft, weil er beispielsweise seine Position über einen längeren Zeitraum nicht mehr verändert hat. In vorteilhafter Weiterbildung ist zur Bestimmung des Sauerstoffgehaltes des Blutes des Patienten ein entsprechender Sensor vorhanden.

In einer bevorzugten Ausgestaltung ist der nasale Applikator als Nasenmaske ausgebildet, die vom Patienten in an sich gängiger Weise am Kopf angebracht werden kann. Der nasale Applikator kann auch in Form von Nasenkanülen ausgebildet sein.

Eine vorteilhafte Weiterbildung sieht vor, dass an dem Therapiegerät ein passiver Flussbegrenzer vorgesehen ist.

Ferner ist vorteilhaft eine Atemflusskontrolle, in Form eines Pneumotachographen oder Spirometers vorgesehen.

In weiterer vorteilhafter Ausbildung ist eine Erfassungseinrichtung vorhanden. So kann die Atmung und die verabreichte Aerosolmenge überwacht und dokumentiert werden. Zusätzlich lassen sich diese Daten in telemedizinische Konzepte einbinden. In vorteilhafter Ausführung kann die Regelung der Aerosolmenge auch über eine Rückkopplung bestimmter Parameter der Atmung oder anderer Parameter ergänzt werden (z. B. bei Verwendung der Sauerstoffsättigung).

Das Therapiegerät zur Steuerung nasaler Aerosolinhalation in die Atemwege, insbesondere die Lunge, eines Patienten weist somit im Wesentlichen eine Beatmungsvorrichtung auf, die steuerbar ist, um den Atemwegen des Patienten über einen Beatmungsschlauch gemäß einem vorgegebenen Atemmuster kontinuierlich Atemluft zuzuführen. Außerdem ist ein regelbarer Aerosolerzeuger, vorzugsweise als Aerosolvernebler ausgeführt, zum Zuführen von Aerosolpartikeln in den im Beatmungsschlauch fließenden Atemluftstrom vorgesehen. Eine ebenso vorgesehene Steuerungseinrichtung ist derart eingerichtet, den Aerosolvernebler gemäß einem Aktivierungsmuster zu aktivieren, das auf das Atemmuster abgestimmt ist, so dass jeweils ein Aerosolbolus gemäß regelmäßigen, ganzzahligen Vielfachen des Atemmustertaktes erzeugt wird.

Besonders vorteilhaft wird die Erfindung in einem Verfahren zur nasalen Aerosolinhalation in die Atemwege, insbesondere die Lunge, eines Patienten, verwendet, wobei vorzugsweise folgende Schritte vorgesehen sind:
Bereitstellen eines Beatmungsschlauchs, über den der Patient nasal ein- und ausatmen kann;
Zuführen von Aerosolpartikeln mittels eines Aerosolverneblers in den im Beatmungsschlauch fliesenden Atemluftstrom, wobei der Aerosolvernebler Aerosolpartikel mit einer Größe von 0,1 bis 3 µm gemäß einem vorgegebenen Aktivierungsmuster erzeugt, wobei der Inhalationsfluss während der Aerosolpartikelzufuhr auf 500 ml/s begrenzt wird; und wobei das Inhalationsvolumen während der Aerosolpartikelzufuhr auf mehr als 0,3 l eingestellt wird.

Gemäß einem weiteren Aspekt stellt die Erfindung ein Verfahren zur nasalen Aerosolinhalation in die Atemwege, insbesondere die Lunge eines Patienten, mit den Schritten bereit:
Bereitstellen einer Beatmungsvorrichtung, die steuerbar ist, um den Atemwegen des Patienten über einen Beatmungsschlauch gemäß einem vorgegebenen Atemmuster diskontinuierlich Atemluft zuzuführen;
Zuführen von Aerosolpartikeln mittels eines Aerosolverneblers in den im Beatmungsschlauch fliesenden Atemluftstrom;
wobei der Aerosolvernebler gemäß einem Aktivierungsmuster aktiviert wird, das auf das Atemmuster abgestimmt ist, so dass jeweils ein Aerosolbolus gemäß regelmäßigen, ganzzahligen Vielfachen des Atemmustertaktes erzeugt wird.

Der erfindungsgemäße Ansatz der nasalen Langzeitinhalation stellt einen Paradigmenwechsel in der Inhalationstechnologie dar. Anstelle der aktiven Kurzzeitinhalation hoher Dosierung am Tage tritt die passive Inhalation mit geringen Dosierungen, die in der Nacht über einen langen Zeitraum erfolgen kann. Die nächtliche Inhalation erfolgt passiv durch Spontanatmung im Schlaf für beispielsweise sechs bis acht Stunden. Alternativ ist erfindungsgemäß aber auch eine Inhalation über eine Nasenkanüle während der Wachphase möglich (Dies ermöglicht eine einfache Gabe eines Aerosols bei bettlägerigen Patienten). Die Erfindung ist also nicht notwendigerweise auf eine Nachtinhalation beschränkt, wenngleich dies jedoch bevorzugt ist.

Die Inhalationstherapie war bisher immer an eine aktive Mitarbeit des Patienten gebunden. Die Applikation der Wirkstoffe erfolgte nur punktuell in starken Dosierungen, was die Gefahr schädlicher Nebenwirkungen erhöht. Die Lungenperipherie wird außerdem mit den bekannten Inhalationsmethoden oft nicht oder nur unzureichend erreicht. Der erfindungsgemäße Ansatz ist auch sehr gut geeignet für die Kinderheilkunde. Dies vor allem, um mögliche Begleiteffekte hoher Dosen der Therapeutika zu minimieren.

Die Erfindung ist vorteilhaft, weil sich dadurch Dosierung und Wirkung der Medikation verbessern lassen, bei einer gleichzeitigen Verringerung der Nebenwirkungen. Es lassen sich gleichzeitig unterschiedliche Wirkstoffe mit einem individuellen Dosis- und Zeitprofil an einem definierten Wirkort applizieren. Vorzugsweise werden Bronchodilatatoren, anti-Inflammatorische, antibakterielle und mukolytische Wirkstoffe verwendet.

Mithilfe der nasalen Inhalation in Kombination mit einer nichtinvasiven Beatmung kann eine deutlich verbesserte Deposition inhalativ applizierbarer Medikamente erzielt werden. Durch den über die nichtinvasive Beatmung induzierten Staudruck soll es zu einer pneumatischen Öffnung/Schienung der kleinen Atemwege kommen, um somit den eigentlichen Wirkort, die Lungenperipherie, besser zu erreichen. Bei der COPD kommt es pathophysiologisch durch die entzündlichen Veränderungen der kleinen Atemwege zu einer Obstruktion derselben. Damit ist der gewünschte therapeutische Effekt mit konventionellen medikamentösen Inhalationsverfahren nur bedingt möglich. Gängige Medikamente (Betamimetika, Steroide) erreichen aufgrund der Obstruktion oft nicht die peripheren Atemwege. Die Beschwerden treten gehäuft nachts und besonders in den frühen Morgenstunden auf. Durch eine geeignete Rückkopplung im System (z. B. Pulsoxymetrie) kann individuell auf eine obstruktive Veränderung reagiert werden. So kann zum Beispiel die Gabe von kurzwirksamen Betamimetika erhöht werden, falls die Sauerstoff-Sättigung einen Schwellenwert unterschreitet. Parallel kann auch die Atmungsunterstützung (z. B. nichtinvasive BiPAP-Therapie) erhöht werden.

Durch das neue technische Verfahren zur zeitgesteuerten Regulierung der nasalen inhalativen Applikation ist eine individualisierte Langzeit-Inhalationstherapie mit einem individuellen Dosisprofil und/oder einem individuellem Zeitprofil möglich. Es können verschiedene Medikamente in definierter Konzentration zu definierten Zeitpunkten und definierten, individuellen Wirkorten verabreicht werden.

Bevorzugte Ausführungsformen der Erfindung umfassen Kombinationen aus erzeugter Partikelgröße, Inhalationsfluss während der Aerosolpartikelzufuhr und Inhalationsvolumen während der Aerosolpartikelzufuhr wie sie in Tabelle 1 angegeben sind.

Besonders bevorzugt ist eine Ausführungsform, bei der der Aerosolerzeuger oder Aerosolvernebler Aerosolpartikel mit einer Größe von 1,25 bis 1,35 µm, insbesondere 1,3 µm erzeugt und der Prozessor den Inhalationsfluss während der Aerosolpartikelzufuhr auf 200 ml/s begrenzt und das Inhalationsvolumen während der Aerosolpartikelzufuhr auf 1,5 l einstellt.

Gemäß einer weiter bevorzugten Ausgestaltung ist der Aerosolvernebler derart eingerichtet, dass Aerosolpartikel mit einer Größe von 1,25 bis 1,35 µm, insbesondere 1,3 µm erzeugt werden und der Prozessor eingerichtet ist, den Inhalationsfluss während der Aerosolpartikelzufuhr auf kleiner als 300 ml/s zu begrenzen und das Inhalationsvolumen während der Aerosolpartikelzufuhr auf 0,5 l einzustellen.

**Tabelle 1**

| | Partikelgröße (µm) | Inhalationsfluss (ml/s) | Inhalationsvolumen (I) |
|---|---|---|---|
| 1 | 0,1 - 3 | < 500 | > 0,3 |
| 2 | 1 - 2 | < 500 | > 0,3 |
| 3 | 1,25 | < 500 | > 0,3 |
| 4 | 1,30 | < 500 | > 0,3 |
| 5 | 1,35 | < 500 | > 0,3 |
| 6 | 0,1 - 3 | < 300 | > 0,3 |
| 7 | 1 - 2 | < 300 | > 0,3 |
| 8 | 1,25 | < 300 | > 0,3 |
| 9 | 1,30 | < 300 | > 0,3 |
| 10 | 1,35 | < 300 | > 0,3 |
| 11 | 0,1 - 3 | < 200 | > 0,3 |
| 12 | 1 - 2 | < 200 | > 0,3 |
| 13 | 1,25 | < 200 | > 0,3 |
| 14 | 1,30 | < 200 | > 0,3 |
| 15 | 1,35 | < 200 | > 0,3 |
| 16 | 0,1 - 3 | < 500 | 0,4 - 2 |
| 17 | 1 - 2 | < 500 | 0,4 - 2 |
| 18 | 1,25 | < 500 | 0,4 - 2 |
| 19 | 1,30 | < 500 | 0,4 - 2 |
| 20 | 1,35 | < 500 | 0,4 - 2 |
| 21 | 0,1-3 | < 300 | 0,4 - 2 |
| 22 | 1 - 2 | < 300 | 0,4 - 2 |
| 23 | 1,25 | < 300 | 0,4 - 2 |
| 24 | 1,30 | < 300 | 0,4 - 2 |
| 25 | 1,35 | < 300 | 0,4 - 2 |
| 26 | 0,1 - 3 | < 200 | 0,4 - 2 |
| 27 | 1 - 2 | < 200 | 0,4 - 2 |
| 28 | 1,25 | < 200 | 0,4 - 2 |
| 29 | 1,30 | < 200 | 0,4 - 2 |
| 30 | 1,35 | < 200 | 0,4 - 2 |
| 31 | 0,1 - 3 | < 500 | 1,5 |
| 32 | 1 - 2 | < 500 | 1,5 |
| 33 | 1,25 | < 500 | 1,5 |
| 34 | 1,30 | < 500 | 1,5 |
| 35 | 1,35 | < 500 | 1,5 |
| 36 | 0,1 - 3 | < 300 | 1,5 |
| 37 | 1 - 2 | < 300 | 1,5 |
| 38 | 1,25 | < 300 | 1,5 |
| 39 | 1,30 | < 300 | 1,5 |
| 40 | 1,35 | < 300 | 1,5 |
| 41 | 0,1 - 3 | < 200 | 1,5 |
| 42 | 1 - 2 | < 200 | 1,5 |
| 43 | 1,25 | < 200 | 1,5 |
| 44 | 1,30 | < 200 | 1,5 |
| 45 | 1,35 | < 200 | 1,5 |

Die Erfindung wird nachstehend unter Bezugnahme auf die beigefügten Zeichnungen näher erläutert. Es zeigen:
- FIG. 1: in schematischer Darstellung ein Therapiegerät, und
- FIG. 2: in Form eines Diagramms die erzielte Lungendeposition für zwei Beispiele.

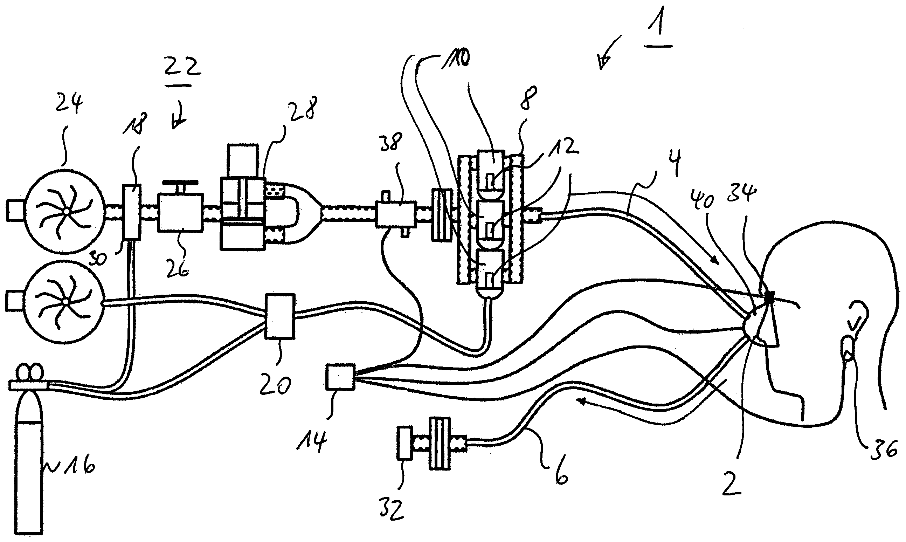

In FIG. 1 ist eine schematische Darstellung eines Therapiegeräts 1 mit einem nasalen Inhalator dargestellt, der im Ausführungsbeispiel als Atem- oder Nasenmaske 2 ausgeführt ist. An die Nasenmaske 2, die bei Bedarf mit einem Verbindungsstück, beispielsweise einem so genannten Y-Konnektor versehen sein kann, sind ein Beatmungsschlauch 4 - zur Zuführung von mit Medikamenten beaufschlagter Atemluft - und ein weiterer, zum Beatmungsschlauch 4 parallel geschalteter Schlauch 6 - zur Ermöglichung einer Ausatmung unter Umgehung des Beatmungsschlauchs 4 - angeschlossen. Somit weist der Beatmungsschlauch 4 die Funktion eines Einatemschlauchs zur Inhalation auf, während der zweite Schlauch 6 als Ausatemschlauch bei der Expiration dient. In die Nasenmaske 2 ist ein geeignetes Umschaltventil oder In-/Expirationsventil, hier nicht näher dargestellt, integriert, das abhängig vom Atemzyklus des Patienten die Schlauchzugänge geeignet verschließt oder freigibt, damit der Patient nicht in den Beatmungsschlauch 4 ausatmet oder umgekehrt.

An der Nasenmaske 2 ist zusätzlich eine nicht näher dargestellte Masken-Druckmessvorrichtung zur Erfassung des Masken- oder Atemdrucks vorgesehen.

An den Beatmungsschlauch 4 ist eingangsseitig ein Aerosolerzeuger 8 angeschlossen. Dieser umfasst eine Anzahl von Medikamentenbehältern 10, in denen jeweils ein gewisser Medikamentenvorrat vorgehalten werden kann. Insbesondere können dabei auch verschieden Medikamente in verschiedenen Behältern vorgehalten und später bedarfs- oder therapieabhängig geeignet miteinander kombiniert werden. Jeder Medikamentenbehälter 10 ist jeweils mit einem Aerosolvernebler 12 versehen, der aus dem im jeweiligen Behälter vorgehaltenen Medikament geringe Mengen an Aerosol erzeugt und der zur Zuführung in den Beatmungsschlauch 4 vorgesehenen Atemluft für den Patienten beimischt. Anstelle eines Verneblers kann dabei jeweils auch ein anderer geeigneter Aerosloerzeuger vorgesehen sein. Zusätzlich kann der Medikamentenbehälter durch einen hier nicht dargestellten Medikamentenaufbereiter ergänzt werden.

Der Beatmungsschlauch 4, der den Aerosolerzeuger 8 mit der Nasenmaske 2 verbindet, ist hinsichtlich seiner Dimensionierung, also insbesondere hinsichtlich seiner Länge und seiner Querschnittsabmessungen, derart gewählt, dass das von ihm gebildete Innenvolumen die Übernahme der Funktionalität eines so genannten Spacers ermöglicht. Demzufolge ist das Innenvolumen derart geeignet gewählt, dass die darin befindliche Atemluftmenge, insbesondere auch nach der Beladung mit dem Aerosol, ausreichend groß bemessen ist, so dass eine geeignete Abstimmung mit dem Atemvolumen des Patienten während eines Atemzuges erreichbar ist.

Die Aerosolvernebler 12 und damit auch der Aerosolerzeuger 8 insgesamt sind mit einer gemeinsamen Steuerungseinheit 14 verbunden. Diese steuert über geeignete, in der Figur aus Gründen der Übersicht nicht explizit dargestellte Signalleitungen die Aerosolvernebler 12 individualisiert an. Diese Ansteuerung erfolgt für jeden Medikamentenbehälter 10 bzw. jeden Aerosolvernebler 12 nach einem vorgegebenen Aktivierungsmuster, um Aerosolpartikel gewünschter Größe und auch gewünschter Zusammensetzung oder Mischung entsprechend dem Atem- und/oder Zeitprofil für den Patienten in den Beatmungsschlauch 4 einzubringen.

Dabei ist der oder jeder Aerosolvernebler 12 insbesondere derart ausgebildet, dass er Aerosolpartikel mit einer Größe von 0,1 bis 3 µm erzeugt. Die geeignete Partikelgröße wird durch die Verwendung spezieller Filter innerhalb des jeweiligen Aerosolverneblers 12, hier nicht näher dargestellt, oder direkt über einen speziellen Aerosolerzeuger, ebenfalls nicht dargestellt, generiert.

Insbesondere durch die Mehrzahl der Medikamentenbehälter 10 mit den entsprechenden Aerosolverneblern 12 kann auch eine Kombination einzelner Medikamente für die Inhalation aufbereitet und geeignet bereitgestellt werden. So kann bedarfsgerecht und auf den Patienten abgestimmt das notwendige Medikament über den Aerosolvernebler 12 in den Beatmungsschlauch 4 zur Inhalation vernebelt werden. Zusätzlich ist ein Sauerstoffreservoir 16 sowohl eingangsseitig über einen Mischer 18 an den Beatmungsschlauch 4 als auch über ein Verteilerventil 20 an den Aerosolerzeuger 8 bzw. individuell an alle oder einige der Medikamentenbehälter 10 angeschlossen, um bedarfsgerecht bei der Beatmung des Patienten und/oder bei der Aerosolerzeugung zusätzlichen Sauerstoff bereitstellen zu können.

Um die Verabreichung der Medikamente gegebenenfalls und bedarfsgerecht aktiv unterstützen zu können, umfasst das Therapiegerät 1 zudem eine eingangsseitig an den Beatmungsschlauch 4 angeschlossene Beatmungsvorrichtung 22. Diese ist im Ausführungsbeispiel insbesondere als BiPAP- und/oder CPAP-System ausgetaltet und umfasst einen Blower 24, ein BiPAP- und/oder CPAP-Ventil 26, ein so genanntes Resistanz-Ventil 28 und ein nicht näher dargestelltes Sperrventil. Die Steuerungseinheit 14, die auch den bzw. die Aerosolvernebler 12 ansteuert, ist dabei signalseitig ebenfalls mit diesen Komponenten verbunden.

Während der Aerosolpartikelzufuhr begrenzt die Steuerungseinheit 14 einerseits den Inhalationsfluss auf 500 ml/s und stellt andererseits das Inhalationsvolumen auf mehr als 0,3 l ein.

Die steuerbare Beatmungsvorrichtung 22 führt, insbesondere mittels des BiPAP- und/oder CPAP-Ventils 26, gemäß einem vorgegebenen Atemmuster während der Einatmung des Patienten dem Beatmungsschlauch 4 kontinuierlich Atemluft, ggf. mit Überdruck, zu. Die Beatmungsvorrichtung 22 erkennt dabei über geeignete Sensoren und eine entsprechende Signalauswertung die Eigenatmung des Patienten und den Atemzyklus und verstärkt diese, um ein ausreichendes Atemvolumen zu gewährleisten. Um dabei eine umfassende Signalauswertung und eine für eine Vielzahl von Situationen geeignete Ansteuerung des Systemkomponenten zu ermöglichen, sind dabei als Sensoren insbesondere ein Sauerstoffsensor 30, ein PEEP-Motor 32 (angeschlossen an das Ende des Schlauchs 6), ein Lagesensor 34, ein Pulsoximeter 36 und Sensoren 38, 40 zur Fluss-/Druckmessung im Beatmungsschlauch 4 vorgesehen.

Das der Beatmungsvorrichtung 22 vorgegebene Atemmuster ist vorzugsweise abgestimmt auf den Zielort der Aerosoldeposition in der Lunge des Patienten. Der applizierte Überdruck erzeugt eine pneumatische Schienung der kleinen Atemwege und hält diese so offen, um so vor allem einen Zugang in obstruktive Bereiche der peripheren Lunge zu ermöglichen.

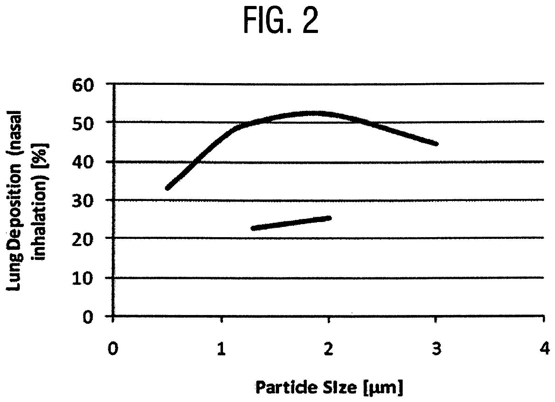

FIG. 2 zeigt anhand einer bevorzugten erfindungsgemäßen Ausführungsform das Ergebnis der Lungendeposition mit einem Therapiegerät 1 zur nasalen Inhalation über eine Nasenmaske 2 oder Nasenkanülen. Bei dem hier verwendeten Therapiegerät 1 wurde der regelbare Aerosolvernebler 8 derart eingerichtet, dass Aerosolpartikel mit einer Größe im Mittel von 1,3 µm erzeugt wurden. Die Steuerungseinrichtung 18 hat den Inhalationsfluss während der Aerosolpartikelzufuhr auf 200 ml/s begrenzt und das Inhalationsvolumen während der Aerosolpartikelzufuhr betrug 1,5 l. Damit wurde eine Lungendeposition von 50 % des Wirkstoffes erreicht.

Bei gleicher Partikelgröße, einem auf 300 ml/s erhöhten Fluss, aber auf 500 ml reduziertem Volumen beträgt die Lungendeposition 25 %, wie ebenfalls in FIG. 2 gezeigt ist.

### Bezugszeichenliste

- 1: Therapiegerät
- 2: Nasenmaske
- 4: Beatmungsschlauch
- 6: Schlauch
- 8: Aerosolerzeuger
- 10: Medikamentenbehälter
- 12: Aerosolvernebler
- 14: Steuerungseinheit
- 16: Sauerstoffreservoir
- 18: Mischer
- 20: Verteilerventil
- 22: Beatmungsvorrichtung
- 24: Blower
- 26: BiPAP-Ventil
- 28: Resistanz-Ventil
- 30: Sauerstoffsensor
- 32: PEEP-Motor
- 34: Lagesensor
- 36: Pulsoximeter
- 38, 40: Sensor zur Fluss-/Druckmessung

## Patentansprüche

1. Therapiegerät (1) zur Verwendung zur Verabreichung von Medikamenten durch nasale Inhalation, mit einem Beatmungsschlauch (4), an den endseitig ein nasaler Applikator und eingangsseitig eine Anzahl von Medikamentenbehältern (10) und ein Aerosolerzeuger (8) angeschlossen sind, und mit einer Steuerungseinheit (14), die den Aerosolerzeuger (8) nach einem vorgegebenen Aktivierungsmuster aktiviert, **dadurch gekennzeichnet, dass** die Steuerungseinheit (14) den Inhalationsfluss während der Aerosolpartikelzufuhr auf höchstens 500 ml/s begrenzt und das Inhalationsvolumen auf mindestens 0,3 l einstellt.

2. Therapiegerät (1) nach Anspruch 1, **dadurch gekennzeichnet, dass** der Aerosolerzeuger (8) Aerosolpartikel mit einer Größe von 0,1 bis 3 µm erzeugt.

3. Therapiegerät (1) nach Anspruch 1 oder 2 **dadurch gekennzeichnet, dass** eine steuerbare Beatmungsvorrichtung (22) vorgesehen ist.

4. Therapiegerät (1) nach Anspruch 3, **dadurch gekennzeichnet, dass** die Beatmungsvorrichtung (22) gemäß einem vorgegebenen Atemmuster während der (Ein)Atmung kontinuierlich Sauerstoff und/oder Atemluft zuführt.

5. Therapiegerät (1) nach Anspruch 4, **dadurch gekennzeichnet, dass** das der Beatmungsvorrichtung (22) vorgegebene Atemmuster abgestimmt ist auf den Zielort der Aersoldeposition im Nasen-Rachen-Raum und/oder der Lunge.

6. Therapiegerät (1) nach einem der vorherigen Ansprüche, **dadurch gekennzeichnet, dass** die Steuerungseinrichtung (14), abgestimmt auf das Atemmuster, den Aerosolerzeuger (8) gemäß einem Aktivierungsmuster aktiviert und jeweils einen Aerosolbolus gemäß regelmäßigen, ganzzahligen Vielfachen des Atemmustertaktes erzeugt.

7. Therapiegerät (1) nach einem der vorherigen Ansprüche, **dadurch gekennzeichnet, dass** die Steuerungseinrichtung (14) über den Aerosolerzeuger (8) während der Expiration des Patienten den Beatmungsschlauch (4) mit Aerosol befüllt, vorzugsweise im Sinne eines Spacers.

8. Therapiegerät (1) nach einem der vorherigen Ansprüche, **dadurch gekennzeichnet, dass** die Steuerungseinrichtung (14) derart ausgelegt ist, dass die Inhalationsdosis einem individuell für den Patienten und der Medikation festgelegtem Zeitprofil folgt.

9. Therapiegerät (1) nach einem der vorherigen Ansprüche, **dadurch gekennzeichnet, dass** ein Sensor für die Lagebestimmung des Patienten vorhanden ist.

10. Therapiegerät (1) nach einem der vorherigen Ansprüche, **dadurch gekennzeichnet, dass** ein Sensor für die Bestimmung des Sauerstoffgehaltes des Blutes des Patienten vorhanden ist.

11. Therapiegerät (1) nach einem der vorherigen Ansprüche, **dadurch gekennzeichnet, dass** eine Atemflusskontrolle und/oder Atemdruckkontrolle vorgesehen ist.

12. Therapiegerät (1) nach einem der vorherigen Ansprüche, **dadurch gekennzeichnet, dass** eine Datenerfassungs- und/oder Datenübertragungsvorrichtung vorhanden ist.

## Claims

1. Therapy appliance (1) for use in administering medication by nasal inhalation, comprising a breathing tube (4), to the end of which a nasal applicator is connected and to the input end of which a number of medication containers (10) and an aerosol generator (8) are connected, and comprising a control unit (14) which activates the aerosol generator (8) according to a predetermined activation pattern, **characterised in that** the control unit (14) limits the inhalation flow during the supply of aerosol particles to at most 500 ml/s and sets the inhalation volume to at least 0.3 I.

2. Therapy appliance (1) according to claim 1, **characterised in that** the aerosol generator (8) generates aerosol particles of from 0.1 to 3 µm in size.

3. Therapy appliance (1) according to either claim 1 or claim 2, **characterised in that** a controllable breathing device (22) is provided.

4. Therapy appliance (1) according to claim 3, **characterised in that** the breathing device (22) continually supplies oxygen and/or breathable air according to a predetermined breathing pattern during an (intake of) breath.

5. Therapy appliance (1) according to claim 4, **characterised in that** the breathing pattern predetermined by the breathing device (22) is adapted to the target location of the aerosol deposition in the nasopharynx and/or the lungs.

6. Therapy appliance (1) according to any of the preceding claims, **characterised in that** the control apparatus (14), in a manner adapted to the breathing pattern, activates the aerosol generator (8) according to an activation pattern and generates in each case an aerosol bolus according to regular, integral multiples of the breathing pattern cycle.

7. Therapy appliance (1) according to any of the preceding claims, **characterised in that** the control apparatus (14) fills the breathing tube (4) with aerosol by means of the aerosol generator (8) while the patient exhales, preferably in the sense of a spacer.

8. Therapy appliance (1) according to any of the preceding claims, **characterised in that** the control apparatus (14) is designed such that the inhalation dose follows a time profile that has been individually determined for the patient and the medication.

9. Therapy appliance (1) according to any of the preceding claims, **characterised in that** a sensor for determining the position of the patient is provided.

10. Therapy appliance (1) according to any of the preceding claims, **characterised in that** a sensor for determining the oxygen content of the blood of the patient is provided.

11. Therapy appliance (1) according to any of the preceding claims, **characterised in that** a means for controlling breathing flow and/or breathing pressure is provided.

12. Therapy appliance (1) according to any of the preceding claims, **characterised in that** a data collection device and/or data transmission device is provided.

## Revendications

1. Appareil de thérapie (1) utilisé pour administrer des médicaments par inhalation nasale, comportant un tuyau de ventilation (4), sur lequel sont raccordés, sur son extrémité, un applicateur nasal et, au niveau de son entrée, un certain nombre de récipients à médicaments (10) et un générateur d'aérosol (8), et comportant une unité de contrôle (14), qui active le générateur d'aérosol (8) d'après un modèle d'activation prédéterminé, **caractérisé en ce que** l'unité de contrôle (14) limite le débit d'inhalation à un maximum de 500 ml/s lors de l'apport des particules d'aérosol et définit le volume d'inhalation à au moins 0,3 l.

2. Appareil de thérapie (1) selon la revendication 1, **caractérisé en ce que** le générateur d'aérosol (8) génère des particules d'aérosol dont la taille est de 0,1 à 3 µm.

3. Appareil de thérapie (1) selon la revendication 1 ou 2, **caractérisé en ce qu'**il est prévu un dispositif respiratoire (22) contrôlable.

4. Appareil de thérapie (1) selon la revendication 3, **caractérisé en ce que** le dispositif respiratoire (22) alimente en continu en oxygène et/ou en air respirable au cours de la respiration/inhalation, selon un modèle respiratoire prédéfini.

5. Appareil de thérapie (1) selon la revendication 4, **caractérisé en ce que** le modèle respiratoire défini pour dispositif respiratoire (22) concorde avec le lieu de destination du dépôt de l'aérosol dans le rhinopharynx et/ou dans les poumons.

6. Appareil de thérapie (1) selon l'une des revendications précédentes, **caractérisé en ce que** le dispositif de contrôle (14), concordant avec le modèle respiratoire, active le générateur d'aérosol (8) conformément à un modèle d'activation et génère respectivement un bolus d'aérosol conformément à un multiple régulier et entier du cycle de modèle respiratoire.

7. Appareil de thérapie (1) selon l'une des revendications précédentes, **caractérisé en ce que** le dispositif de contrôle (14) remplit le tuyau respiratoire (4) avec de l'aérosol lors de l'expiration du patient, par l'intermédiaire du générateur d'aérosol (8), de préférence dans le sens d'un espaceur.

8. Appareil de thérapie (1) selon l'une des revendications précédentes, **caractérisé en ce que** le dispositif de contrôle (14) est dimensionné de telle sorte que la dose d'inhalation respecte un profil de temps défini individuellement pour le patient et la médication.

9. Appareil de thérapie (1) selon l'une des revendications précédentes, **caractérisé en ce qu'**un capteur est prévu pour déterminer la position du patient.

10. Appareil de thérapie (1) selon l'une des revendications précédentes, **caractérisé en ce qu'**il est prévu un capteur pour déterminer le taux d'oxygène dans le sang du patient.

11. Appareil de thérapie (1) selon l'une des revendications précédentes, **caractérisé en ce qu'**il est prévu un contrôle du débit respiratoire et/ou un contrôle de la pression respiratoire.

12. Appareil de thérapie (1) selon l'une des revendications précédentes, **caractérisé en ce qu'**il est prévu un dispositif de saisie de données et/ou un dispositif de transmission des données.
